(19) Europäisches Patentamt
European Patent Office
Office européen des brevets

(11) EP 1 911 435 B1

(12) **EUROPEAN PATENT SPECIFICATION**

(45) Date of publication and mention of the grant of the patent:
**24.07.2013 Bulletin 2013/30**

(51) Int Cl.:
***A61K 8/03*** *(2006.01)* ***A61K 8/58*** *(2006.01)*
***A61K 8/81*** *(2006.01)* ***A61Q 5/06*** *(2006.01)*
***A61K 8/41*** *(2006.01)*

(21) Application number: **06021171.1**

(22) Date of filing: **10.10.2006**

(54) **Two-phase composition for improving curl retention**

Kosmetische Zwei-Phasen-Zusammensetzungen zur Verbesserung des Erhalts von Locken

Composition cosmétique biphase pour améliorer la tenue des boucles

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**16.04.2008 Bulletin 2008/16**

(73) Proprietor: **Kao Germany GmbH**
**64297 Darmstadt (DE)**

(72) Inventors:
- **Bräutigam, Ina**
  **64297 Darmstadt (DE)**
- **Förster, Sabine**
  **64319 Pfungstadt (DE)**

(56) References cited:
**WO-A-02/060397** **DE-A1- 19 703 475**
**US-A1- 2002 031 478**

Remarks:
The file contains technical information submitted after the application was filed and not included in this specification

EP 1 911 435 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention is related to a composition for conditioning and improving curl retention of curly hair comprising two optically separated phases when standing at zero shear rate which becomes homogeneous upon shaking.

Conditioning compositions have been known for many years. Among them it is possible to find products on hair care market for conditioning and especially improving curl retention of curly hair. On the other hand, there are products on hair care market for conditioning and styling hair.

For people having curly hair it is important that the curls do appear nice for a long time. With nice curly hair appearance it is meant that curls are neatly distributed over the hair length. Within the meaning of the present description, with the term curly hair it is meant that the curly hair can be natural or permanently shaped or curled with a hot iron or with curling rod.

WO 02/060397 discloses dual phase conditioning and styling compositions for heat styling hair. According to the document the compositions comprise an oil phase comprising volatile oil and an aqueous alcoholic phase comprising a styling polymer, an emulsifier and a salt. The document does not address at all treatment of curly hair, let alone curl retention of curly hair.

EP 996 408 discloses dual phase compositions for make up cleansing wherein a polyvinylpyrrolidone copolymer is used as a demixing agent. The document does not address at all hair care application of such composition.

The inventors of the present invention have surprisingly found out that a two phase composition comprising an oil phase and a water phase wherein two phases are optically separated at a zero shear rate and become homogeneous upon shaking and return again to optically separated two phases upon release of agitation improves curl retention of curly hair.

Accordingly, the object of the present invention is a composition for hair comprising 5 to 50%, by weight calculated to the total composition, of oil phase and 50 to 95%, by weight calculated to the total composition, of aqueous phase with a pH between 3 and 7 wherein the oil phase comprises at least one volatile oil at a concentration of 70 to 100%, by weight, calculated to the content of the oil phase wherein the volatile oil is dimethicone and the aqueous phase comprises 0.1 to 20% by weight calculated to the content of the aqueous phase at least one nonionic fixing polymer, and at least one cationic fixing polymer 10 to 40% by weight calculated to the content of the aqueous phase at least one water miscible organic solvent and 0.05 to 2% by weight calculated to the content of the aqueous phase at least one cationic surfactant wherein the oil and aqueous phases are optically separated at a zero shear rate and becomes homogeneous upon shaking and returns again to optically separated two phases upon release of agitation.

Further objective of the present invention is the use of the composition comprising 5 to 50%, by weight calculated to the total composition of oil phase and 50 to 95%, by weight calculated to the total composition, of aqueous phase with a pH between 3 and 7 wherein the oil phase comprises at least one volatile oil at a concentration of 70 to 100%, by weight, calculated to the content of the oil phase wherein the volatile oil is dimethicone, and the aqueous phase comprises 0.1 to 10% by weight calculated to the content of the aqueous phase at least one nonionic fixing polymer and at least one cationic fixing polymer 10 to 40% by weight calculated to the content of the aqueous phase at least one water miscible organic solvent and 0.05 to 2% by weight calculated to the content of the aqueous phase at least one cationic surfactant wherein the oil and aqueous phases are optically separated at a zero shear rate and become homogeneous upon shaking and return again to optically separated two phases upon release of agitation for improving curl retention of curly hair.

The composition of the present invention comprises optically separated two phases which become homogeneous upon shaking and return again to optically separated two phases upon release of agitation. The composition of the present invention returns to optically separated two phases upon release of agitation preferably within 24 hours, more preferably within 20 hours and most preferably within 10 hours. It should be noted that with the composition of the present invention separated oil and aqueous phases are recognizable after 15 to 30 minutes of shaking. The above referred separation periods refer to the complete separation of the two phases i.e. returning to their original state.

The composition of the present invention comprises oil phase at a concentration of 5 to 50%, preferably 10 to 40% more preferably 15 to 30% by weight calculated to total composition. The oil phase comprises 70 to 100%, preferably 80 to 100% and more preferably 90 to 100% by weight calculated to the content of the oil phase at least one volatile oil. Suitable volatile dimethicone.

Suitable volatile dimethicones are the ones with a viscosity of less than 5 $mm^2/s$. The most preferred are dimethicone with a viscosity of 1 $mm^2/s$ and 0.65 $mm^2/s$ which are available from Dow Corning with the trade name DC 200 Fluid.

Non-volatile silicones may also be incorporated into the compositions of the present invention at a low concentration, i.e. lower than 1% by weight calculated to the content of oil phase. Suitable ones are arylated silicones such as phenyltrimethicone known with the trade name Dow Corning 556, and dimethicones with higher viscosity.

In addition, natural oils such as olive oil, almond oil, avocado oil, wheatgerm oil, ricinus oil can be incorporated into the oil phase. The concentration of natural oils is below 1 %, preferably below 0.5% by weight calculated to the

content of the oil phase. Solubility of the natural oil in used volatile oil should be examined in selecting natural oil. As a rule selected natural oil should be soluble in the volatile oil used in the composition.

The compositions of the present invention comprises aqueous phase at a concentration of 50 to 95%, preferably 60 to 90% more preferably 70 to 85% by weight calculated to the total composition. The aqueous phase comprises 0.1 to 20%, preferably 0.5 to 15%, more preferably 0.5 to 10 % by weight calculated to the content of the aqueous phase of non ionic and cationic fixing polymers. In selecting the fixing polymers attention should be paid to the solubility of the polymer in aqueous phase as described above and also in the total composition wherein oil and aqueous phases are present together and mixed to become homogeneous composition by mixing.

Anionic polymers may also be used so far if no incompatibility is observed in the presence of cationic surfactant. One of the important point in selecting fixing polymer is that the polymer should not thicken the aqueous phase. In other words the viscosity of the aqueous phase should remain low i.e. below 100 mPa.s, preferably 50 mPa.s.

Suitable non-ionic polymers are polyvinylpyrrolidone, polyvinylpyrrolidone/vinylacetate copolymer and polyvinylpyrrolidone/vinylacetate/vinylpropionate copolymer. Preferred is polyvinylpyrrolidone/vinylacetate copolymer. In addition to these fixing polymers derivatives of natural polymers such as hydroxyl ethylcellulose may also be used in combination with one of the fixing polymers mentioned above.

Suitable cationic polymers include Polyquaternium-4, Polyquaternium-11, Polyquaternium-16, Polyquaternium-18, Polyquaternium-24, Polyquaternium-28, Polyquaternium-46, polyvinylpyrrolidone/dimethylaminoethyl methacrylate, and cationically derivatized natural polymers. Preferred are Polyquaternium-4, Polyquaternium-11 and Polyquaternium-16.

Suitable amphoteric polymers which may be used are those available from the company National Starch under the trade name Amphomer such as Octylacrylamide/Acrylates/Butylaminoethyl methacrylate copolymer, those available under the trade name Diaformer (methacryloylethylbetaine/methacrylates copolymer). Preferred are the ones available under the trade name Amphomer.

The aqueous phase of the composition comprises combination of non-ionic and cationic fixing polymers.

As a fixing polymer silicone containing polymers either grafted or block copolymer are also useful. Suitable and the preferred one is Polysilicone-9.

The aqueous phase of the composition of the present invention comprises 10 to 40%, preferably 15 to 35%, more preferably 20 to 30% by weight calculate to the content of aqueous phase at least one water miscible organic solvent-. With the term "water miscible" it is meant that the organic solvent is miscible with water at any ratio. Suitable ones are $C_2$ - $C_4$ monohydric alcohols such as ethanol, propanol, isopropanol, butanol and isobutanol, benzyl alcohol and benzyloxyethanol and their mixtures. Most preferred are ethanol and isopropanol.

The aqueous phase of the composition of the present invention comprises at least one cationic surfactant at a concentration of 0.05 to 2%, preferably 0.1 to 1%, more preferably 0.2 to 0.75% by weight calculated to the content of the aqueous phase. Suitable cationic surfactants are according to the general formula

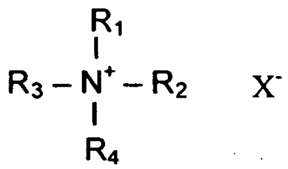

where $R_1$ is a saturated or unsaturated, branched or non-branched alkyl chain with 8-22 C atoms or

$R_5$ CO NH $(CH_2)_n$

where $R_5$ is saturated or unsaturated, branched or non-branched alkyl chain with 7-21 C atoms and n has typical value of 0 - 4 or

$R_6$ CO O $(CH_2)_n$

where $R_6$ is saturated or unsaturated, branched or non-branched alkyl chain with 7-21 C atoms and n has typical value of 0 - 4, and
$R_2$ is a saturated or unsaturated, branched or non-branched alkyl chain with 1-22 C atoms or

$R_5$ CO NH $(CH_2)_n$

where $R_5$ is a saturated or unsaturated, branched or non-branched alkyl chain with 7-21 C atoms and n has typical value of 0 - 4 or

$$R_6\ CO\ O\ (CH_2)_n$$

where $R_6$ is a saturated or unsaturated, branched or non-branched alkyl chain with 7-21 C atoms and n has typical value of 0 - 4,

and $R_3$ and $R_4$ are independent from each other lower alkyl chain with 1 to 4 carbon atoms, and X is chloride, bromide or methosulfate.

Suitable cationic surfactants are particularly cetyl trimethly ammonium chloride, steartrimonium chloride, behentrimoinium chloride, stearamidopropyl trimonuim chloride, behenamidopropylethyldimonium ethosulfate, behenamidopropyltrimonium methosulfate, cocamidopropyltrimonium chloride, cocotrimonim chloride, palmitamidopropyltrimonum chloride, dipalmitoyltrimonium chloride, di-C12-C15 alkyldimoniumchloride, distearyldimonium chloride, dipalmitoylethylhydroxyethylmonium chloride, dioleoylethyl dimethyl ammonium methosulfate, dioleoylethyl hydroxyethylmonium methosulfate, dilinolamidopropyldimonium chloride, dioleylethyl hydroxyethylmonium chloride and dipalmitoylethyldimonium chloride.

In another embodiment the aqueous phase comprises a mixture of two cationic surfactants such as steartrimonium chloride and di-C12-C15 alkyldimoniumchloride.

The pH of the aqueous phase varies from 3 to 7, particularly 4.5 to 6. For adjusting the pH of the said conditioner compositions, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, pyruvic acid, salicylic acid or acetic acid or inorganic acids such as hydrochloric acid, phosphoric acid, sulphuric acid, nitric acid. The pH of the conditioner composition can also be adjusted to the required pH by using alkaline solution such as sodium hydroxide, potassium hydroxide, ammonium hydroxide or their salts with those acids mentioned above.

The composition of the present invention can comprise UV filters for protection of hair from environmental influences. It should be noted that oil soluble UV filters are contained in the oil phase and water soluble ones are added to the aqueous phase although partitioning the UV filter between two phases is not excluded. The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxybenzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher and/or 2-ethylhexyl-2-cyano-3,3-diphenyl acrylate also known as Octocrylene.

The preferred amount of the UV-absorber ranges from about 0.01 % to 2.5%, more preferably from 0.05 % to 1 % by weight, calculated to the total composition.

The moisturising agents may be included into the aqueous phase of the composition. Suitable ones are panthenol, polyols, such as glycerol, polyethylene glycols with molecular weight 200 to 20,000. The moisturising ingredients can be included in the aqueous phase of the compositions at a concentration range of 0.01 - 2.5% by weight calculated to the content of the aqueous phase.

Natural plant extracts may as well form part of the composition of the present invention. Natural extracts should be included into the aqueous phase of the compositions. Suitable aqueous (e.g. steam-distilled) alcoholic or hydro-alcoholic plant extracts known **per se** are in particular extracts from leaves, fruits, blossoms, roots, rinds or stems of almond, aloe, pineapple, artichoke, arnica, avocado, valerian, henbane, birch, stinging nettle, echinacea, ivy, wild angelica, gentian, ferns, pine needles, silver weed, ginseng, broom, bamboo, green tea, oat, rose hip, hamamelis, hay flowers, elderberry, hop, coltsfoot, currants, chamomile, carrots, chestnuts, clover, burr root, coconut, cocoanut, mango, peach, lemon, cornflower, wheat, apricot, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

The aqueous and oil phases of the composition of the present invention may be coloured. The dyestuff suitable for product colouring purposes are all useful for this purpose. It should be noted that for colouring aqueous phase water soluble dyes should be used and for colouring oil phase oil soluble dyes are suitable. The nature of the dyestuff is actually not important but preferred are non substantive dyes, i.e. not remaining on hair after washing with water or shampooing.

Fragrance, chelating agent, preservatives and other conventional cosmetic ingredients can be included at their

usual concentrations either into the oil or aqueous phases depending on their solubility.

The composition of the present invention is preferably transparent, i.e. both oil and aqueous phases are transparent as judged by a naked eye in a transparent glass or PET bottle with a thickness of up to 3 cm.

The composition of the present invention is a leave-in composition, i.e. not rinsed off from the hair after application. Accordingly, in a process the composition of the present invention is applied to the shampooed and/or wetted hair and the hair is dried. It is possible to apply the composition of the present invention as a lotion and also from a bottle equipped with a pump spray. Preferred application is spraying onto hair.

Following examples are to illustrate the invention but not to limit

**Example 1** (reference example)

Oil phase

| | % by weight |
|---|---|
| Trisiloxane | 99.8 |
| Fragrance | 0.2 |

Aqueous phase

| | % by weight |
|---|---|
| VP/VA copolymer | 3.5 |
| Steartrimoniumchloride | 0.2 |
| Di-$C_{12}$-$C_{15}$ alkyldimonium chloride | 0.1 |
| Ethanol | 23.0 |
| Citric acid/Ammonium hydroxide | q.s. to pH 6.0 |
| Water | to 100 |

The above composition is transparent . The above composition is used at a concentration of oil phase of 15% by weight and 85% aqueous phase.

Curl retention of a curly hair tress curled using a rod with a diameter of 1.1 cm was determined with the above composition as follows. The hair tress used was approximately 3g and having a length of 20 cm before curling. The length of curled hair was 12 cm. To the towel dried hair approximately 0.5 g of the above composition was applied with by spraying after homogeneously mixing both phases by shaking and the tress was dried with a hair drier. Curl retention was determined under ambient conditions at a temperature of approximately 25°C and at a relative humidity of approximately 60%. At predetermined time intervals the length of hair was measured and curl retention was calculated using the following equation.

$$\text{Curl retention \%} = ((L - L_t) / (L - L_0)) \times 100$$

wherein L is the original length of hair streak, $L_t$ is the length of curled hair at time t and $L_0$ is the length of curled at the beginning of the test.

For comparative purposes similar hair tress was used but not treated with the above composition.

Curl retention %

| Time (min) | Example 1 | Comparative |
|---|---|---|
| 0 | 100 | 100 |

(continued)

| Time (min) | Example 1 | Comparative |
|---|---|---|
| 5 | 98 | 97 |
| 10 | 96 | 90 |
| 15 | 95 | 81 |
| 20 | 92 | 76 |
| 25 | 90 | 71 |
| 30 | 89 | 68 |
| 35 | 87 | 64 |
| 40 | 84 | 63 |
| 45 | 83 | 61 |
| 50 | 81 | 58 |
| 55 | 79 | 54 |
| 60 | 77 | 53 |

From the above results it is clear that Example 1 improves curl retention.

**Example 2**

Oil phase

| | % by weight |
|---|---|
| Trisiloxane | 99.8 |
| Fragrance | 0.2 |

Aqueous phase

| | % by weight |
|---|---|
| VP/VA copolymer | 3.5 |
| Polyquaternium-11 | 0.5 |
| Steartrimoniumchloride | 0.15 |
| Di-C12-C15 alkyldimonium chloride | 0.15 |
| Ethanol | 20.0 |
| Lactic acid/Ammonium hydroxide | q.s. to pH 6.0 |
| Water | to 100 |

**Example 3**

Oil phase

| | % by weight |
|---|---|
| Dimethicone 1 cst | 99.8 |
| Fragrance | 0.2 |

Aqueous phase

| | % by weight |
|---|---|
| VP/VA copolymer | 3.0 |
| Polysilicone-9 | 0.5 |
| Cetrimonium chloride | 0.15 |
| Dioleylethyl hydroxyethylmonium chloride | 0.15 |
| Benzophenone-4 | 0.10 |
| Ethanol | 25.0 |
| Lactic acid/Ammonium hydroxide | q.s. to pH 6.0 |
| Dyestuff CI 19140 and CI 42053 | q.s. |
| Water | to 100 |

**Example 4**

Oil phase

| | % by weight |
|---|---|
| Dimethicone 1 cst | 99.6 |
| Benzophenone-3 | 0.2 |
| Fragrance | 0.2 |

Aqueous phase

| | % by weight |
|---|---|
| VP/VA copolymer | 3.0 |
| Polysilicone-9 | 0.5 |
| Cetrimonium chloride | 0.15 |
| Dioleylethyl hydroxyethylmonium chloride | 0.15 |
| Benzophenone-4 | 0.10 |
| Ethanol | 25.0 |
| Lactic acid/Ammonium hydroxide | q.s. to pH 7.0 |
| Dyestuff CI 19140 and CI 42053 | q.s. |
| Water | to 100 |

## Claims

1. A two phase composition for hair **characterised in that** it comprises 5 to 50%, by weight calculated to the total composition, of an oil phase and 50 to 95%, by weight calculated to the total composition, of an aqueous phase with a pH between 3 and 7, wherein the oil phase comprises at least one volatile oil at a concentration of 70 to 100%, by weight, calculated to the content of the oil phase, whereby volatile oil is a dimethicone, and the aqueous phase comprises 0.1 to 20% by weight calculated to the content of the aqueous phase, of at least one nonionic fixing polymer and at least one cationic fixing polymer, 10 to 40% by weight calculated to the content of the aqueous phase of at least one water miscible organic solvent and 0.05 to 2% by weight calculated to the content of the aqueous phase at least one cationic surfactant, wherein the oil and aqueous phases are optically separated at a zero shear rate and become homogeneous upon shaking and return again to optically separated two phases at a zero shear rate.

2. The composition according to claim 1 **characterised in that** the cationic surfactant in aqueous phase is selected from compounds according to general formula

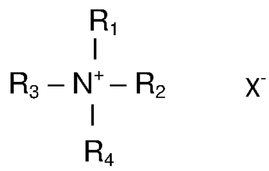

where $R_1$ is a saturated or unsaturated, branched or non-branched alkyl chain with 8-22 C atoms or

$R_5$ CO NH $(CH_2)_n$

where $R_5$ is a saturated or unsaturated, branched or non-branched alkyl chain with 7-21 C atoms and n has typical value of 0 - 4 or

$R_6$ CO O $(CH_2)_n$

where $R_6$ is a saturated or unsaturated, branched or non-branched alkyl chain with 7-21 C atoms and n has typical value of 0 - 4, and
$R_2$ is a saturated or unsaturated, branched or non-branched alkyl chain with 1-22 C atoms or

$R_5$ CO NH $(CH_2)_n$

where $R_5$ is a saturated or unsaturated, branched or non-branched alkyl chain with 7-21 C atoms and n has typical value of 0 - 4 or

$R_6$ CO O $(CH_2)_n$

where $R_6$ is a saturated or unsaturated, branched or non-branched alkyl chain with 7-21 C atoms and n has typical value of 0 - 4,
and $R_3$ and $R_4$ are independent from each other lower alkyl chain with 1 to 4 carbon atoms, and X is chloride, bromide or methosulfate.

3. The composition according to any of the preceding claims **characterised in that** it comprises at least one UV filter either in the oil or in the aqueous phase.

4. The composition according to any of the preceding claims **characterised in that** it is transparent.

5. Use of the composition according to any of the preceding claims for improving curl retention of hair.

6. Process for improving curl retention of hair **characterised in that** a composition according to claims 1 to 4 is applied to wet or shampooed and towel dried hair and without rinsing off hair is dried.

## Patentansprüche

1. Eine Zweiphasen- Zusammensetzung für Haare, **dadurch gekennzeichnet, dass** sie 5-bis 50 Gew.- % Ölphase, berechnet auf die Gesamtzusammensetzung, und 50 bis 95 Gew.- % wässrige Phase, berechnet auf die Gesamtzusammensetzung, mit einem pH- Wert zwischen 3 und 7 enthält, wobei die Ölphase mindestens ein flüchtiges Öl in einer Menge von 70 bis 100 Gew.- % enthält, berechnet auf den Inhalt der Ölphase, und das flüchtige Öl ein Dimethicon ist und die wässrige Phase mindestens ein nicht ionisches fixierendes Polymer und mindestens ein kationisches fixierendes Polymer in einer Menge von 0,1 bis 20 Gew.- %, berechnet auf den Inhalt der wässrigen Phase, enthält, mindestens ein wassermischbares organisches Lösungsmittel in einer Menge von 10 bis 40 Gew.- %, berechnet auf den Inhalt der wässrigen Phase und mindestens ein kationisches Tensid in einer Menge von 0,05 bis 2 Gew.- % enthält, berechnet auf den Inhalt der wässrigen Phase, wobei die Öl- und die wässrigen Phasen bei einem Null- Schergefälle optisch getrennt sind und durch schütteln homogen werden und optisch wieder separate

zwei Phasen werden bei einem Null-Schergefälle.

2. Zusammensetzung nach Anspruch 1, **dadurch gekennzeichnet, dass** das kationische Tensid in der wässrigen Phase aus Verbindungen entsprechend der allgemeinen Formel

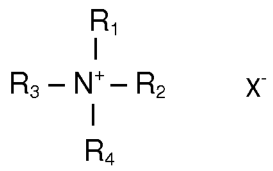

ausgewählt ist,
wo $R_1$ eine gesättigte oder ungesättigte, verzweigte oder nicht verzweigte Alkylkette mit 8-22 C- Atomen ist, oder

$R_5$ CO NH $(CH_2)_n$

wo $R_5$ eine gesättigte oder ungesättigte, verzweigte oder nicht verzweigte Alkylkette mit 7-21 C- Atomen ist und n einen typischen Wert von 0 - 4 hat, oder

$R_6$ CO O $(CH_2)_n$

wo $R_6$ eine gesättigte oder ungesättigte, verzweigte oder nicht verzweigte Alkylkette mit 7-21 C- Atomen ist und n einen typischen Wert von 0 - 4 hat, und
$R_2$ eine gesättigte oder ungesättigte, verzweigte oder nicht verzweigte Alkylkette mit 1-22 C-Atomen ist, oder

$R_5$ CO NH $(CH_2)_n$

wo $R_5$ eine gesättigte oder ungesättigte, verzweigte oder nicht verzweigte Alkylkette mit 7-21 C- Atomen ist und n einen typischen Wert von 0 - 4 hat, oder

$R_6$ CO O $(CH_2)_n$

wo $R_6$ eine gesättigte oder ungesättigte, verzweigte oder nicht verzweigte Alkylkette mit 7-21 C- Atomen ist, und n einen typischen Wert von 0 - 4 hat,
und $R_3$ und $R_4$ unabhängig voneinander H oder niedrige Alkylketten mit 1 bis 4 Kohlenstoffatomen sind, und X Chlorid, Bromid oder Methosulfat ist.

3. Zusammensetzung nach einem der vorhergehenden Ansprüche, **dadurch gekennzeichnet, dass** sie mindestens einen UV- Filter, entweder in der Öl- oder in der wässrigen Phase, enthält.

4. Zusammensetzung nach einem der vorhergehenden Ansprüche **dadurch gekennzeichnet, dass** sie transparent ist.

5. Verwendung der Zusammensetzung nach einem der vorhergehenden Ansprüche zur Verbesserung der Bewahrung des Wellbildes von Haaren.

6. Verfahren zur Verbesserung zur Bewahrung des Wellbildes von Haaren, **dadurch gekennzeichnet, dass** eine Zusammensetzung nach den Ansprüchen 1 bis 4 auf feuchtes oder shampooniertes, handtuchtrockenes Haar aufgetragen wird und ohne auszuspülen das Haar getrocknet wird.

## Revendications

1. Une composition pour cheveux en deux phases **caractérisée par le fait que** 5 à 50% du poids total calculé par rapport à la composition totale est une phase huileuse et que 50 à 95% du poids total calculé par rapport à la composition totale est une phase aqueuse ayant un pH compris entre 3 et 7, alors que la phase huileuse comprend

au moins une huile volatile à une concentration de 70 à 100% par rapport au poids, calculé par rapport au contenu de la phase huileuse, par quoi l'huile volatile est un diméthicone et la phase aqueuse comprend 0,1 à 20% du poids calculé par rapport au contenu de la phase aqueuse, d'au moins un polymère fixant non-ionique et d'au moins un polymère fixant cationique, 10 à 40% du poids calculé par rapport au contenu de la phase aqueuse d'au moins un solvant organique miscible et 0,05 à 2% du poids calculé par rapport au contenu de la phase aqueuse, d'au moins un surfactant cationique dans quoi les phases huileuse et aqueuse sont visiblement séparées suivant un taux de cisaillement nul et deviennent homogènes lorsque agitées et retournent à un état où elles forment deux phases séparées suivant un taux de cisaillement nul.

2. La composition, en accord avec la déclaration 1, se **caractérise par le fait que** le surfactant cationique dans la phase aqueuse est sélectionné à partir de composés suivant la formule

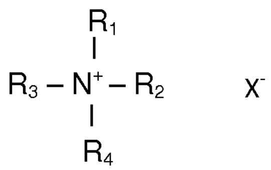

où $R_1$ est une chaine alcane saturée ou insaturée, ramifiée ou non-ramifiée avec 8-22 atomes C ou

$R_5$ CO NH $(CH_2)_n$

où $R_5$ est une chaine alcane saturée ou insaturée, ramifiée ou non-ramifiée avec 7-21 atomes C et a une valeur typique de 0 - 4 ou

$R_6$ CO O $(CH_2)_n$

où $R_6$ est une chaine alcane saturée ou insaturée, ramifiée ou non-ramifiée avec 7-21 atomes C et n a une valeur typique de 0 - 4 et
$R_2$ est une chaine alcane saturée ou insaturée, ramifiée ou non-ramifiée avec 1-22 atomes C ou

$R_5$ CO NH $(CH_2)_n$

où $R_5$ est une chaine alcane saturée ou insaturée, ramifiée ou non-ramifiée avec 7-21 atomes C et a une valeur typique de 0 - 4 ou

$R_6$ CO O $(CH_2)_n$

où $R_6$ est une chaine alcane saturée ou insaturée, ramifiée ou non-ramifiée avec 7-21 atomes C et n a une valeur typique de 0 - 4,
et $R_3$ et $R_4$ sont indépendants de leurs H respectifs ou de leurs chaines alcanes ramifiées de 1 - 4 atomes de carbones, et X est un atome de chlore, brome ou methosulfate.

3. La composition, en accord avec les déclarations précédentes, se **caractérise par le fait qu'**elle comprend au moins un filtre UV dans l'une des deux phases, aqueuse ou huileuse.

4. La composition, en accord avec les déclarations précédentes, se **caractérise par le fait qu'**elle est transparente.

5. L'utilisation de la composition, en accord avec chacune des déclarations précédentes, a pour but d'améliorer la tenue des boucles sur les cheveux.

6. Le processus permettant d'améliorer la tenue des boucles de cheveux se **caractérise par le fait que** la composition, en accord avec les déclarations 1 à 4, est appliquée sur des cheveux mouillés ou lavés et essorés et les cheveux sont séchés ensuite sans être rincés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02060397 A
- EP 996408 A